# EUROPÄISCHE PATENTSCHRIFT

(11) **EP 2 509 935 B1**
(45) Veröffentlichungstag und Bekanntmachung des Hinweises auf die Patenterteilung: **16.10.2013**
(21) Anmeldenummer: 10787752.4
(22) Anmeldetag: 06.12.2010
(51) Int. Cl.: C07C 5/29, C07C 13/18

(54) **VERFAHREN ZUR ISOMERISIERUNG EINES GESÄTTIGTEN, VERZWEIGTEN UND ZYKLISCHEN KOHLENWASSERSTOFFS**
METHOD FOR ISOMERIZING A SATURATED, BRANCHED, AND CYCLICAL HYDROCARBON
PROCÉDÉ D'ISOMÉRISATION D'UN HYDROCARBURE SATURÉ, RAMIFIÉ ET CYCLIQUE

(30) Priorität: 07.12.2009 EP 09178208
(43) Veröffentlichungstag der Anmeldung: 17.10.2012
(73) Patentinhaber: BASF SE, 67056 Ludwigshafen (DE)
(72) Erfinder: TSCHIRSCHWITZ, Steffen, 68199 Mannheim (DE); DEUERLEIN, Stephan, 67061 Ludwigshafen (DE); BÜRKLE, Jochen, 68163 Mannheim (DE); SCHMITT, Markus, 69115 Heidelberg (DE); SCHREIBER, Michael, 68167 Mannheim (DE); OEHLENSCHLÄGER, Steffen, B-2050 Antwerpen (BE)
(86) Internationale Anmeldenummer: PCT/EP2010/068958
(87) Internationale Veröffentlichungsnummer: WO 2011/069957

(56) Entgegenhaltungen:
- EP-A2- 1 346 768
- VLADISLAV A KSENOFONTOV ET AL: "Isomerization of cyclic hydrocarbons mediated by an AlCl3-based ionic liquid as catalyst", REACTION KINETICS AND CATALYSIS LETTERS, SPRINGER SCIENCE+BUSINESS MEDIA, DORDRECHT, NL, Bd. 80, Nr. 2, 1. November 2003 (2003-11-01), Seiten 329-335, XP019265121, ISSN: 1588-2837 in der Anmeldung erwähnt
- NOBUMASA RITAJIMA, YOSHIO ONO: "Cu( AlCl)4 as a catalyst for the isomerisation of pentane at room temperature", JOURNAL OF MOLECULAR CATALYSIS, 1. Januar 1981 (1981-01-01), Seiten 121-122, XP002616940, lausanne

## Beschreibung

Die Erfindung betrifft ein Verfahren zur Isomerisierung eines gesättigten, verzweigten und zyklischen Kohlenwasserstoffs, wobei bei der Isomerisierung ein tertiäres C-Atom des Kohlenwasserstoffs in ein sekundäres C-Atom umgewandelt wird.

Die Isomerisierung von gesättigten Kohlenwasserstoffen (Paraffinen) zu den korrespondierenden verzweigten Isomeren ist ein wichtiger Prozess, um z. B. die Research Octanzahl (ROZ) von Benzin zu erhöhen, um dessen Verbrennungseigenschaften zu verbessern.

Verzweigte zyklische Kohlenwasserstoffe können unter Ringerweiterung zu weniger verzweigten zyklischen Kohlenwasserstoffen isomerisieren; ein Beispiel ist die Umlagerung von Methylcyclopentan (MCP) zu Cyclohexan. Katalysiert werden diese Reaktionen durch starke Lewis-Säuren bzw. starke Brønsted-Säuren.

Die Isomerisierung gesättigter Kohlenwasserstoffe mit festem Aluminiumchlorid ist seit langem bekannt. Als beschleunigender Zusatz wird häufig HCl eingesetzt (z. B. US 2,493,567, US 3,233,001, US 5,202,519). Problematisch in diesen Verfahren ist die Langzeitstabilität von festem Aluminiumchlorid und dessen Abtrennung.

US 2003/0109767 A1 (Vasina et al.) berichtet, dass ionische Flüssigkeiten bestehend aus einem stickstoffhaltigen heterocyclischen oder aliphatischen Kation und einem Anion, das sich von einem Metallhalogenid ableitet, zur Isomerisierung von Paraffinen in Richtung höher verzweigter Paraffine bei relativ niedrigen Temperaturen genutzt werden können.

Zyklische Kohlenwasserstoffe mit einem tertiären Kohlenstoffatom als Additive, wie Methylcyclohexan und Dimethylcyclopentan, erhöhen gemäß EP 1 403 236 A1 (Haldor Topsoe A/S) die Selektivität bezüglich der Bildung stärker verzweigter Kohlenwasserstoffe aus weniger oder nicht verzweigten Kohlenwasserstoffen.

Ionische Flüssigkeiten bestehend aus n-Butylpyridiniumchlorid und Aluminiumchlorid können benutzt werden, um Methylcyclopentan und Cyclohexan zu isomerisieren: V.A. Ksenofontov, T.V. Vasina, Y.E. Zubarev, L.M. Kustov, React. Kinet. Catal. Lett. 2003, Vol. 80 (2), Seiten 329-335.

Die Nutzung von Kombinationen aus Aluminiumhalogeniden und Kupfer(II)-chlorid oder Kupfer(II)-sulfat jeweils im Verhältnis 1:1 für die Isomerisierung von Pentan wird von Ono et al. in Chem. Lett. 1978, 1061-64; J. Catal. 1979, 56, 47-51; und J. Catal. 1980, 64, 13-17, berichtet.

Die Isomerisierung von Paraffinen mit Aluminiumchlorid und Kupfer(II)-chlorid in molaren Verhältnissen von 2:1 bis 3:1 wird in US 5,202,519 A (Phillips Petroleum Comp.) beschrieben, wobei die Selektivität bezüglich der Isomensierung geringer ist als die Selektivität bezüglich Disproportionierung.

In US 2003/0181780 A1 (Herbst et al.) wird über die Isomerisierung von Paraffinen zu höher verzweigten Kohlenwasserstoffen mit ionischen Flüssigkeiten, die Aluminiumhalogenid und zusätzlich Metallhalogenide, wie z. B. Kupfer-(II)-chlorid, Eisen-(III)-chlorid und Molybdän-(V)-chlorid, enthalten, berichtet.

Der vorliegenden Erfindung lag die Aufgabe zugrunde, unter Überwindung von Nachteilen des Stands der Technik, ein verbessertes wirtschaftliches Verfahren zur Isomerisierung eines gesättigten, verzweigten und zyklischen Kohlenwasserstoffs bereitzustellen.
Das Herstellverfahren sollte zudem besonders einfach und wirtschaftlich sein und das Verfahrensprodukt (ein gesättigter, zyklischer Kohlenwasserstoff mit gleicher Summenformel und geringerem Verzweigungsgrad) in hohen Ausbeuten, insbesondere in hohen Raum-Zeit-Ausbeuten (RZA), und Selektivitäten liefern.

Demgemäß wurde ein Verfahren zur Isomerisierung eines gesättigten, verzweigten und zyklischen Kohlenwasserstoffs, wobei bei der Isomerisierung ein tertiäres C-Atom des Kohlenwasserstoffs in ein sekundäres C-Atom umgewandelt wird, gefunden, welches dadurch gekennzeichnet ist, dass man die Isomerisierung in Gegenwart einer super-sauren ionischen Flüssigkeit, umfassend ein organisches Kation und ein anorganisches Anion, wobei das Anion ein super-saures Aluminiumtrichlorid-Lewisbase-Addukt ist, und 0,1 bis 1,5 Gew.-% einer Kupfer(II)-Verbindung bezogen auf die eingesetzte super saure ionische Flüssigkeit durchführt.

Erfindungsgemäß wurde erkannt, dass die Isomerisierung eines gesättigten, verzweigten und zyklischen Kohlenwasserstoffs stark beschleunigt und die Selektivität erhöht werden kann, wenn die Isomerisierung in Gegenwart einer super-sauren ionischen Flüssigkeit enthaltend Aluminiumchlorid und in Gegenwart einer Kupfer(II)-Verbindung als Katalysator durchgeführt wird.
Somit ist das erfindungsgemäße Verfahren herkömmlichen Verfahren überlegen, u. a. weil die Reaktionsgleichgewichte deutlich schneller erreicht werden.

Bei der Kupfer(II)-Verbindung handelt es sich bevorzugt um ein Kupfer(II)-Salz.

Bevorzugte Kupfer(II)-Verbindungen sind CuCl₂, CuO, CuSO₄, Cul₂, CuBr₂, Cu(OH)Cl, Cu(HSO₄)₂, Cu(NO₃)₂.

Besonders bevorzugt handelt es sich bei der Kupfer(II)-Verbindung um CuCl₂, CuO oder CuSO₄.

Besonders bevorzugt wird das erfindungsgemäße Isomerisierungsverfahren in Gegenwart einer Kupfer(II)-Verbindung in Abwesenheit einer weiteren Verbindung eines anderen Metalls, insbesondere eines weiteren Halogenids eines anderen Metalls, durchgeführt, wobei es sich bei dem Metall besonders um in Nebengruppen-Metall handelt.

Die Isomerisierung wird in Gegenwart von 0,1 bis 1,5 Gew.-%, insbesondere > 0,1 bis 1,5 Gew.-%, ganz besonders 0,5 bis 1,5 Gew.=%, der Kupfer(II)-Verbindung, jeweils bezogen auf die eingesetzte super-saure ionische Flüssigkeit, durchführt.

Bei dem zu isomerisierenden Kohlenwasserstoff handelt es sich bevorzugt um einen gesättigten, verzweigten und zyklischen C₄₋₁₈-Kohlenwasserstoff, besonders um einen gesättigten, verzweigten und zyklischen C₅₋₁₀-Kohlenwasserstoff, ganz besonders um einen gesättigten, verzweigten und zyklischen C₅₋₈-Kohlenwasserstoff.

Besonders bevorzugt handelt es sich bei dem zu isomerisierenden Kohlenwasserstoff um Methylcyclopentan (MCP), das zum Produkt Cyclohexan (CH) isomerisiert wird, und/oder um 1,2-Dimethylcyclopentan,1,3-Dimethylcyclopentan und/oder 1,1-Dimethylcyclopentan, die zum Produkt Methylcyclohexan (MCH) isomerisiert werden.

Mit dem erfindungsgemäßen Isomerisierungsverfahren wird ein tertiäres C-Atom des Kohlenwasserstoffs in ein sekundäres C-Atom umgewandelt. Die Isomerisierung liefert also einen weniger verzweigten gesättigten Kohlenwasserstoff als Produkt. Besonderes Beispiel hiefür ist die Isomerisierung von Methylcyclopentan (MCP) zu Cyclohexan (CH).

Der zu isomerisierende Kohlenwasserstoff wird bevorzugt in einer Konzentration im Bereich von 1 bis 90 Gew.-%, besonders von 5 bis 20 Gew.-%, jeweils bezogen auf die ionische Flüssigkeit, einsetzt.

Die Isomerisierung wird bevorzugt bei einer Temperatur im Bereich von -20 bis 150 °C, besonders 40 bis 100 °C, durchführt.

Die Isomerisierung wird bevorzugt bei einem Absolutdruck im Bereich von 1 bis 10 bar, besonders 1 bis 6 bar, durchführt.

Ionische Flüssigkeiten im Sinne der vorliegenden Erfindung sind vorzugsweise Salze der allgemeinen Formel

### (A) Salze der allgemeinen Formel (I)

[A]⁺ₙ [Y]ⁿ⁻ (I),

in der n für 1, 2, 3 oder 4 steht, [A]⁺ für ein quartäres Ammonium-Kation, ein O-xonium-Kation, ein Sulfonium-Kation oder ein Phosphonium-Kation und [Y]ⁿ⁻ für ein ein-, zwei-, drei- oder vierwertiges Anion steht;

### (B) gemischte Salze der allgemeinen Formeln (II)

| | |
|---|---|
| [A1]⁺[A²]⁺ [Y]ⁿ⁻ | (IIa), wobei n = 2; |
| [A¹]⁺[A²]⁺[A³]⁺ [Y]ⁿ⁻ | (IIb), wobei n = 3; oder |
| [A1]⁺[A2]⁺[A³]⁺[A⁴]⁺ [Y]ⁿ⁻ | (IIc), wobei n = 4 und |

wobei [A1]⁺, [A²]⁺, [A³]⁺ und [A⁴]⁺ unabhängig voneinander aus den für [A]⁺ genannten Gruppen ausgewählt sind und [Y]ⁿ⁻ die unter (A) genannte Bedeutung besitzt; oder

### (C) gemischte Salze der allgemeinen Formeln (III)

| | |
|---|---|
| [A¹]⁺[A²]⁺[A³]⁺[M¹]⁺ [Y]ⁿ⁻ | (IIIa), wobei n = 4; |
| [A¹]+[A²]+[M¹]+[M²]⁺ [Y]ⁿ⁻ | (IIIb), wobei n = 4; |
| [A1]⁺[M¹]⁺[M²]⁺[M³]⁺ [Y]ⁿ⁻ | (IIIc), wobei n = 4; |
| [A¹]+[A²]+[M¹]⁺ [Y]ⁿ⁻ | (IIId), wobei n = 3; |
| [A¹]+[M¹]+[M²]⁺ [Y]ⁿ⁻ | (IIIe), wobei n = 3; |
| [A1]⁺[M¹]⁺ [Y]ⁿ⁻ | (IIIf), wobei n = 2; |
| [A¹]+[A²]+[M4]²⁺ [Y]ⁿ⁻ | (IIIg), wobei n = 4; |
| [A¹]⁺[M¹]⁺[M⁴]²⁺ [Y]ⁿ⁻ | (IIIh), wobei n = 4; |
| [A¹]⁺[M⁵]³⁺ [Y]ⁿ⁻ | (Illi), wobei n = 4; oder |
| [A¹]+[M⁴]²⁺ [Y]ⁿ⁻ | (IIIj), wobei n = 3 und |

wobei [A¹]⁺, [A²]⁺ und [A³]⁺ unabhängig voneinander aus den für [A]⁺ genannten Gruppen ausgewählt sind, [Y]ⁿ⁻ die unter (A) genannte Bedeutung besitzt und [M¹]⁺, [M²]⁺, [M³]⁺ einwertige Metallkationen, [M⁴]²⁺ zweiwertige Metallkationen und [M⁵]³⁺ dreiwertige Metallkationen bedeuten.

Vorzugsweise besitzen die ionischen Flüssigkeiten einen Schmelzpunkt von weniger als 180 °C. Weiterhin bevorzugt liegt der Schmelzpunkt in einem Bereich von -50 °C bis 150 °C, mehr bevorzugt im Bereich von -20 °C bis 120 °C und weiterhin mehr bevorzugt unter 100°C.

Bei den erfindungsgemäßen ionischen Flüssigkeiten handelt es sich um organische Verbindungen, d. h. dass mindestens ein Kation oder ein Anion der ionischen Flüssigkeit einen organischen Rest enthält.

Verbindungen, die sich zur Bildung des Kations [A]⁺ von ionischen Flüssigkeiten eignen, sind z. B. aus DE 102 02 838 A1 bekannt. So können solche Verbindungen Sauerstoff-, Phosphor-, Schwefel- oder insbesondere Stickstoffatome enthalten, beispielsweise mindestens ein Stickstoffatom, bevorzugt 1-10 Stickstoffatome, besonders bevorzugt 1-5, ganz besonders bevorzugt 1-3 und insbesondere 1-2 Stickstoffatome. Gegebenenfalls können auch weitere Heteroatome wie Sauerstoff-, Schwefel- oder Phosphoratome enthalten sein. Das Stickstoffatom ist ein geeigneter Träger der positiven Ladung im Kation der ionischen Flüssigkeit, von dem im Gleichgewicht dann ein Proton bzw. ein Alkylrest auf das Anion übergehen kann, um ein elektrisch neutrales Molekül zu erzeugen.

Für den Fall, dass das Stickstoffatom der Träger der positiven Ladung im Kation der ionischen Flüssigkeit ist, kann bei der Synthese der ionischen Flüssigkeiten zunächst durch Quaternisierung am Stickstoffatom etwa eines Amins oder Stickstoff-Heterocyclus' ein Kation erzeugt werden. Die Quaternisierung kann durch Protonierung oder Alkylierung des Stickstoffatoms erfolgen. Je nach verwendetem Alkylierungsreagens werden Salze mit unterschiedlichen Anionen erhalten. In Fällen, in denen es nicht möglich ist, das gewünschte Anion bereits bei der Quaternisierung zu bilden, kann dies in einem weiteren Syntheseschritt erfolgen. Ausgehend beispielsweise von einem Ammoniumhalogenid kann das Halogenid mit einer Lewissäure umgesetzt werden, wobei aus Halogenid und Lewissäure ein komplexes Anion gebildet wird. Alternativ dazu ist der Austausch eines Halogenidions gegen das gewünschte Anion möglich. Dies kann durch Zugabe eines Metallsalzes unter Ausfällung des gebildeten Metallhalogenids, über einen Ionenaustauscher oder durch Verdrängung des Halogenidions durch eine starke Säure (unter Freisetzung der Halogenwasserstoffsäure) geschehen. Geeignete Verfahren sind beispielsweise in Angew. Chem. 2000, 112, S. 3926 - 3945 und der darin zitierten Literatur beschrieben.

Geeignete Alkylreste, mit denen das Stickstoffatom in den Aminen oder Stickstoff-Heterocyclen beispielsweise quaternisiert sein kann, sind C₁-C₁₈-Alkyl, bevorzugt C₁-C₁₀-Alkyl, besonders bevorzugt C₁-C₆-Alkyl und ganz besonders bevorzugt Methyl. Die Alkylgruppe kann unsubstituiert sein oder einen oder mehrere gleiche oder verschiedene Substituenten aufweisen.

Bevorzugt sind solche Verbindungen, die mindestens einen fünf- bis sechsgliedrigen Heterocyclus, insbesondere einen fünfgliedrigen Heterocyclus, enthalten, der mindestens ein Stickstoffatom sowie gegebenenfalls ein Sauerstoff- oder Schwefelatom aufweist, besonders bevorzugt sind solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit zwei Stickstoffatomen. Weiterhin bevorzugt sind aromatische Heterocyclen.

Besonders bevorzugte Verbindungen sind solche, die ein Molgewicht unter 1000 g/mol aufweisen, ganz besonders bevorzugt unter 500 g/mol.

Weiterhin sind solche Kationen bevorzugt, die ausgewählt sind aus den Verbindungen der Formeln (IVa) bis (IVw), 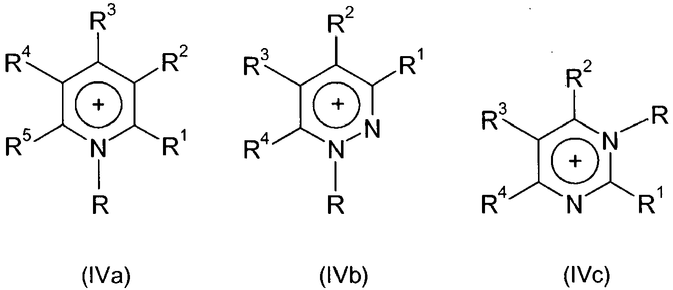 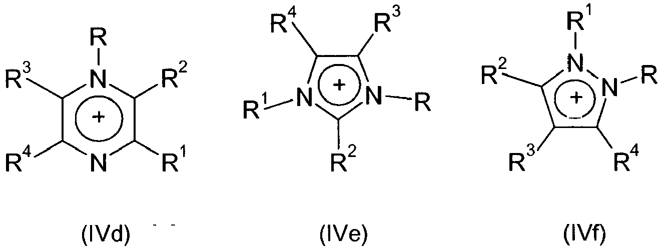 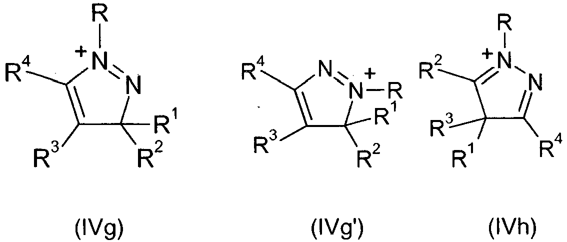 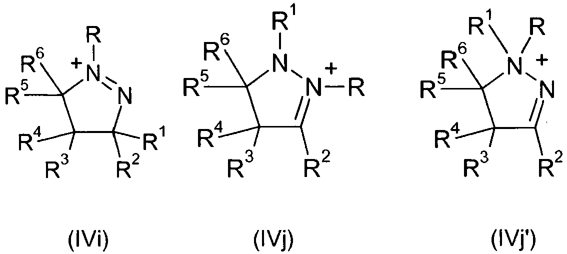 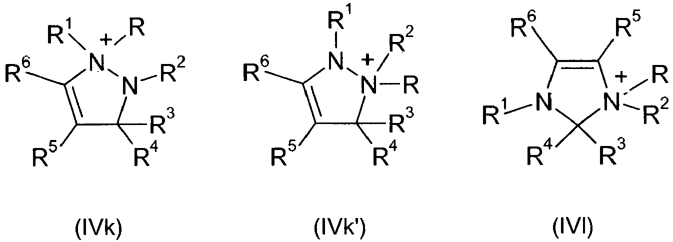 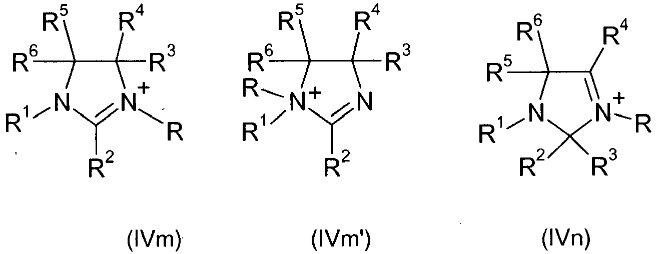 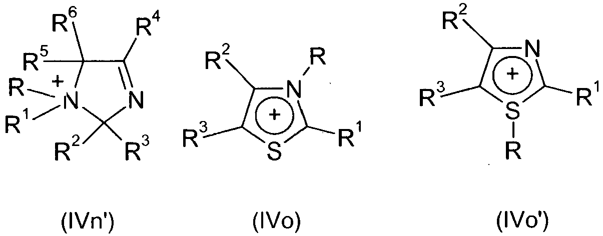 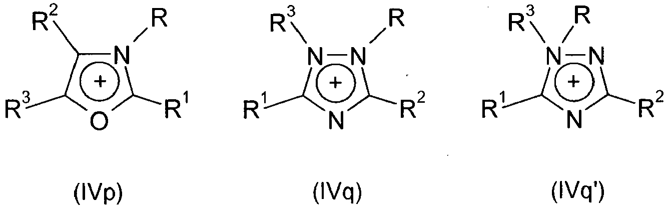 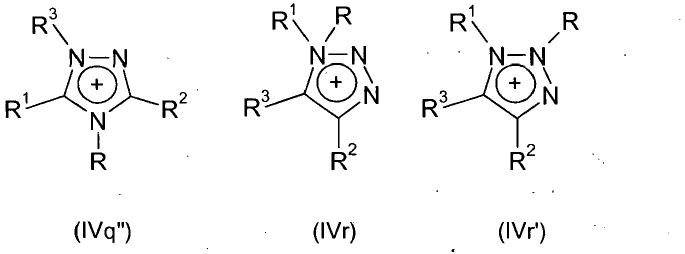 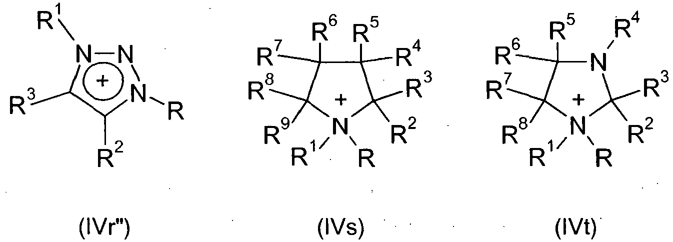 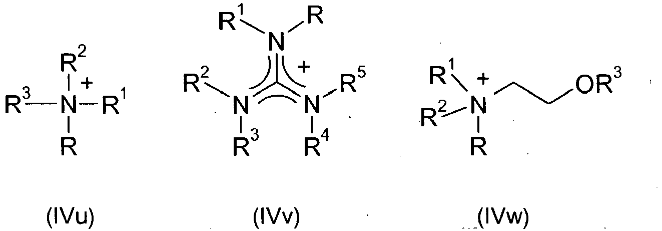 sowie Oligomere, die diese Strukturen enthalten.

Weitere geeignete Kationen sind Verbindungen der allgemeinen Formel (IVx) und (IVy) 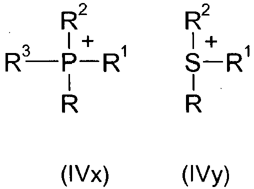 sowie Oligomere, die diese Struktur enthalten.

In den oben genannten Formeln (IVa) bis (IVy) stehen
- der Rest R für Wasserstoff, einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen; und
- die Reste R¹ bis R⁹ unabhängig voneinander für Wasserstoff, eine Sulfo-Gruppe oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 20 Kohlenstoffatomen, wobei die Reste R¹ bis R⁹, welche in den oben genannten Formeln (IV) an ein Kohlenstoffatom (und nicht an ein Heteroatom) gebunden sind, zusätzlich auch für Halogen oder eine funktionelle Gruppe stehen können; oder
   zwei benachbarte Reste aus der Reihe R¹ bis R⁹ zusammen auch für einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen.

Als Heteroatome kommen bei der Definition der Reste R und R¹ bis R⁹ prinzipiell alle Heteroatome in Frage, welche in der Lage sind, formell eine -CH₂-, eine -CH=, eine -C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-, -NR'-, -N=, -PR'-, -PR'₂ und -SiR'₂- genannt, wobei es sich bei den Resten R' um den verbleibenden Teil des Kohlenstoff enthaltenden Rests handelt. Die Reste R¹ bis R⁹ können dabei in den Fällen, in denen diese in den oben genannten Formeln (IV) an ein Kohlenstoffatom (und nicht an ein Heteroatom) gebunden sind, auch direkt über das Heteroatom gebunden sein.

Als funktionelle Gruppen kommen prinzipiell alle funktionellen Gruppen in Frage, welche an ein Kohlenstoffatom oder ein Heteroatom gebunden sein können. Als geeignete Beispiele seien -OH (Hydroxy), =O (insbesondere als Carbonylgruppe), -NH₂ (Amino), -NHR', -NR₂' =NH (Imino), -COOH (Carboxy), -CONH₂ (Carboxamid), -SO₃H (Sulfo) und -CN (Cyano) genannt. Fuktionelle Gruppen und Heteroatome können auch direkt benachbart sein, so dass auch Kombinationen aus mehreren benachbarten Atomen, wie etwa -O- (Ether), -S- (Thioether), -COO- (Ester), -CONH- (sekundäres Amid) oder -CONR'- (tertiäres Amid), mit umfasst sind, beispielsweise Di-(C₁-C₄-Alkyl)-amino, C₁-C₄-Alkyloxycarbonyl oder C₁-C₄-Alkyloxy. Bei den Resten R' handelt es sich um den verbleibenden Teil des Kohlenstoff enthaltenden Restes.

Als Halogene seien Fluor, Chlor, Brom und lod genannt.

Bevorzugt steht der Rest R für
- unverzweigtes oder verzweigtes, unsubstituiertes oder ein bis mehrfach mit Hydroxy, Halogen, Phenyl, Cyano, C₁-C₆-Alkoxycarbonyl und/oder SO₃H substituiertes C₁-C₁₈-Alkyl mit insgesamt 1 bis 20 Kohlenstoffatomen, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl, 1-Octadecyl, Benzyl, 3-Phenylpropyl, 2-Hydroxyethyl, 2-Cyanoethyl, 2-(Methoxycarbonyl)-ethyl, 2-(Ethoxycarbonyl)-ethyl, 2-(n-Butoxy-carbonyl)-ethyl, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl, 6-Hydroxyhexyl und Sulfopropyl;
- Glykole, Butylenglykole und deren Oligomere mit 1 bis 100 Einheiten und einem Wasserstoff oder einem C₁-C₈-Alkyl als Endgruppe, wie beispielsweise R^{A}O-(CHR^{B}-CH₂-O)ₙ-CHR^{B}-CH₂- oder R^{A}O-(CH₂CH₂CH₂CH₂O)ₙ-CH₂CH₂CH₂CH₂O- mit R^{A} und R^{B} bevorzugt Wasserstoff, Methyl oder Ethyl und n bevorzugt 0 bis 3, insbesondere 3-Oxabutyl, 3-Oxapentyl, 3,6-Dioxaheptyl, 3,6-Dioxaoctyl, 3,6,9-Trioxadecyl, 3,6,9-Trioxaundecyl, 3,6,9,12-Tetraoxatridecyl und 3,6,9,12-Tetraoxatetradecyl;
- Vinyl;
- 1-Propen-1-yl, 1-Propen-2-yl und 1-Propen-3-yl; und
- N,N-Di-C₁-C₆-alkyl-amino, wie beispielsweise N,N-Dimethylamino und N,N-Diethylamino.

Besonders bevorzugt steht der Rest R für unverzweigtes und unsubstituiertes C₁-C₁₈-Alkyl, wie beispielsweise Methyl, Ethyl, 1-Propyl, 1-Butyl, 1-Pentyl, 1-Hexyl, 1-Heptyl, 1-Octyl, 1-Decyl, 1-Dodecyl, 1-Tetradecyl, 1-Hexadecyl, 1-Octadecyl, insbesondere für Methyl, Ethyl, 1-Butyl und 1-Octyl sowie für CH₃O-(CH₂CH₂O)ₙ-CH₂CH₂- und CH₃CH₂O-(CH₂CH₂O)ₙ-CH₂CH₂- mit n gleich 0 bis 3.

Bevorzugt stehen die Reste R¹ bis R⁹ unabhängig voneinander für
- Wasserstoff;
- Halogen;
- eine funktionelle Gruppe;
- gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₁-C₁₈-Alkyl;
- gegebenenfalls durch funktionelle Gruppen, Aryl, Alkyl, Aryloxy, Alkyloxy, Halo-gen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₂-C₁₈-Alkenyl;
- gegebenenfalls durch funktionelle Gruppen, Aryl, Alkyl, Aryloxy, Alkyloxy, Halogen, Heteroatome und/oder Heterocyclen substituiertes C₆-C₁₂-Aryl;
- gegebenenfalls durch funktionelle Gruppen, Aryl, Alkyl, Aryloxy, Alkyloxy, Halogen, Heteroatome und/oder Heterocyclen substituiertes C₅-C₁₂-Cycloalkyl;
- gegebenenfalls durch funktionelle Gruppen, Aryl, Alkyl, Aryloxy, Alkyloxy, Halogen, Heteroatome und/oder Heterocyclen substituiertes C₅-C₁₂-Cycloalkenyl; oder
- einen gegebenenfalls durch funktionelle Gruppen, Aryl, Alkyl, Aryloxy, Alkyloxy, Halogen, Heteroatome und/oder Heterocyclen substituierten fünf- bis sechsgliedrigen, Sauerstoff-, Stickstoff- und/oder Schwefelatome aufweisenden Heterocyclus bedeuten; oder
zwei benachbarte Reste zusammen mit den Atomen, an welchen sie gebunden sind, für
- einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring.

Bei gegebenenfalls durch funktionelle Gruppen, Aryl, Alkyl, Aryloxy, Alkyloxy, Halogen, Heteroatome und/oder Heterocyclen substituiertem C₁-C₁₈-Alkyl handelt es sich bevorzugt um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, 2-Etylhexyl, 2,4,4-Trimethylpentyl, 1,1,3,3-Tetramethylbutyl, 1-Nonyl, 1-Decyl, 1-Undecyl, 1-Dodecyl, 1-Tridecyl, 1-Tetradecyl, 1-Pentadecyl, 1-Hexadecyl, 1-Heptadecyl, 1-Octadecyl, Cyclopentylmethyl, 2-Cyclopentylethyl, 3-Cyclopentylpropyl, Cyclohexylmethyl, 2-Cyclohexylethyl, 3-Cyclohexylpropyl, Benzyl (Phenylmethyl), Diphenylmethyl (Benzhydryl), Triphenylmethyl, 1-Phenylethyl, 2-Phenylethyl, 3-Phenylpropyl, α,α-Dimethylbenzyl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, Methoxy, Ethoxy, Formyl, 1,3-Dioxolan-2-yl, 1,3-Dioxan-2-yl, 2-Methyl-1,3-dioxolan-2-yl, 4-Methyl-1,3-dioxolan-2-yl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-di-methylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, Acetyl, CₙF_{2(n-a)+(1-b)}H_{2a+b} mit n gleich 1 bis 30, 0 ≤ a ≤ n und b = 0 oder 1 (beispielsweise CF₃, C₂F₅, CH₂CH₂-C₍ₙ₋₂₎F₂₍ₙ-₂₎₊₁, C₆F₁₃, C₈F₁₇, C₁₀F₂₁, C₁₂F₂₅), Chlormethyl, 2-Chlorethyl, Trichlormethyl, 1,1-Dimethyl-2-chlorethyl, Methoxymethyl, 2-Butoxyethyl, Diethoxymethyl, Diethoxyethyl, 2-Isopropoxyethyl, 2-Butoxypropyl, 2-Octyloxyethyl, 2-Methoxyisopropyl, 2-(Methoxycarbonyl)-ethyl, 2-(Ethoxycarbonyl)-ethyl, 2-(n-Butoxycarbonyl)-ethyl, Butylthiomethyl, 2-Dodecylthioethyl, 2-Phenylthioethyl, 5-Hydroxy-3-oxa-pentyl, 8-Hydroxy-3,6-dioxa-octyl, 11-Hydroxy-3,6,9-trioxaundecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-dioxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxapentadecyl, 9-Methoxy-5-oxa-nonyl, 14-Methoxy-5,10-dioxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxaheptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bei gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₂-C₁₈-Alkenyl handelt es sich bevorzugt um Vinyl, 2-Propenyl, 3-Butenyl, cis-2-Butenyl, trans-2-Butenyl oder CₙF_{2(n-a)-(1-b)}H_{2a-b} mit n≤30, 0 ≤ a ≤ n und b = 0 oder 1.

Bei gegebenenfalls durch funktionelle Gruppen, Aryl, Alkyl, Aryloxy, Alkyloxy, Halogen, Heteroatome und/oder Heterocyclen substituiertes C6-C12-Aryl handelt es sich bevorzugt um Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl; Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2-Nitrophenyl, 4-Nitrophenyl, 2,4-Dinitrophenyl, 2,6-Dinitrophenyl, 4-Dimethylaminophenyl, 4-Acetylphenyl, Methoxyethylphenyl, Ethoxymethylphenyl, Methylthiophenyl, Isopropylthiophenyl oder tert.-Butylthiophenyl oder C6F(5-a)Ha mit 0 ≤ a ≤5.

Bei gegebenenfalls durch funktionelle Gruppen, Aryl, Alkyl, Aryloxy, Alkyloxy, Halogen, Heteroatome und/oder Heterocyclen substituiertes C5-C12-Cycloalkyl handelt es sich bevorzugt um Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl, CnF2(n-a)-(1-b)H2a-b mit n ≤ 30, 0 ≤ a ≤ n und b = 0 oder 1 sowie ein gesättigtes oder ungesättigtes bicyclisches System wie z. B. Norbornyl oder Norbornenyl.

Bei gegebenenfalls durch funktionelle Gruppen, Aryl, Alkyl, Aryloxy, Alkyloxy, Halogen, Heteroatome und/oder Heterocyclen substituiertes C₅-C₁₂-Cycloalkenyl handelt es sich bevorzugt um 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤ 30, 0 ≤ a ≤ n und b = 0 oder 1.

Bei einen gegebenenfalls durch funktionelle Gruppen, Aryl, Alkyl, Aryloxy, Alkyloxy, Halogen, Heteroatome und/oder Heterocyclen substituierten fünf- bis sechsgliedrigen, Sauerstoff-, Stickstoff- und/oder Schwefelatome aufweisenden Heterocyclus handelt es sich bevorzugt um Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl oder Difluorpyridyl.

Bilden zwei benachbarte Reste gemeinsam einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring, so handelt es sich bevorzugt um 1,3-Propylen, 1,4-Butylen, 1,5-Pentylen, 2-Oxa-1,3-propylen, 1-Oxa-1,3-propylen, 2-Oxa-1,3-propylen, 1-Oxa-1,3-propenylen, 3-Oxa-1,5-pentylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Enthalten die oben genannten Reste Sauerstoff- und/oder Schwefelatome und/oder substituierte oder unsubstituierte Iminogruppen, so ist die Anzahl der Sauerstoff- und/oder Schwefelatome und/oder Iminogruppen nicht beschränkt. In der Regel beträgt sie nicht mehr als 5 in dem Rest, bevorzugt nicht mehr als 4 und ganz besonders bevorzugt nicht mehr als 3.

Enthalten die oben genannten Reste Heteroatome, so befinden sich zwischen zwei Heteroatomen in der Regel mindestens ein Kohlenstoffatom, bevorzugt mindestens zwei Kohlenstoffatome.

Besonders bevorzugt stehen die Reste R¹ bis R⁹ unabhängig voneinander für
- Wasserstoff;
- unverzweigtes oder verzweigtes, unsubstituiertes oder ein bis mehrfach mit Hydroxy, Halogen, Phenyl, Cyano, C₁-C₆-Alkoxycarbonyl und/oder SO₃H substituiertes C₁-C₁₈-Alkyl mit insgesamt 1 bis 20 Kohlenstoffatomen, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl, 1-Octadecyl, Benzyl, 3-Phenylpropyl, 2-Hydroxyethyl, 2-Cyanoethyl, 2-(Methoxycarbonyl)-ethyl, 2-(Ethoxycarbonyl)-ethyl, 2-(n-Butoxy-carbonyl)-ethyl, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl, 6-Hydroxyhexyl und Sulfopropyl;
- Glykole, Butylenglykole und deren Oligomere mit 1 bis 100 Einheiten und einem Wasserstoff oder einem C₁- bis C₈-Alkyl als Endgruppe, wie beispielsweise R^{A}O-(CHR^{B}-CH₂-O)ₙ-CHR^{B}-CH₂- oder R^{A}O-(CH₂CH₂CH₂CH₂O)ₙ-CH₂CH₂CH₂CH₂O- mit R^{A} und R^{B} bevorzugt Wasserstoff, Methyl oder Ethyl und n bevorzugt 0 bis 3, insbesondere 3-Oxabutyl, 3-Oxapentyl, 3,6-Dioxaheptyl, 3,6-Dioxaoctyl, 3,6,9-Trioxadecyl, 3,6,9-Trioxaundecyl, 3,6,9,12-Tetraoxatridecyl und 3,6,9,12-Tetraoxatetradecyl;
- Vinyl;
- 1-Propen-1y1, 1-Propen-2-yl und 1-Propen-3y1; und
- N,N-Di-C₁-C₆-alkyl-amino, wie beispielsweise N,N-Dimethylamino und N,N-Diethylamino.

Ganz besonders bevorzugt stehen die Reste R¹ bis R⁹ unabhängig voneinander für Wasserstoff oder C₁-C₁₈-Alkyl, wie beispielsweise Methyl, Ethyl, 1-Butyl, 1-Pentyl, 1-Hexyl, 1-Heptyl, 1-Octyl, für Phenyl, für 2-Hydroxyethyl, für 2-Cyanoethyl, für 2-(Methoxycarbonyl)ethyl, für 2-(Ethoxycarbonyl)ethyl, für 2-(n-Butoxycarbonyl)ethyl, für N,N-Dimethylamino, für N,N-Diethylamino, für Chlor sowie für CH₃O-(CH₂CH₂O)ₙ-CH₂CH₂- und CH₃CH₂O-(CH₂CH₂O)ₙ-CH₂CH₂- mit n gleich 0 bis 3.

Ganz besonders bevorzugt setzt man als Pyridiniumionen (IVa) solche ein, bei denen
- einer der Reste R¹ bis R⁵ Methyl, Ethyl oder Chlor ist und die verbleibenden Reste R¹ bis R⁵ Wasserstoff sind;
- R³ Dimethylamino ist und die verbleibenden Reste R¹, R², R⁴ und R⁵ Wasserstoff sind;
- alle Reste R¹ bis R⁵ Wasserstoff sind;
- R² Carboxy oder Carboxamid ist und die verbleibenden Reste R¹, R², R⁴ und R⁵ Wasserstoff sind; oder
- R¹ und R² oder R² und R³ 1,4-Buta-1,3-dienylen ist und die verbleibenden Reste R¹, R², R⁴ und R⁵ Wasserstoff sind;
und insbesondere solche, bei denen
- R¹ bis R⁵ Wasserstoff sind; oder
- einer der Reste R¹ bis R⁵ Methyl oder Ethyl ist und die verbleibenden Reste R¹ bis R⁵ Wasserstoff sind.

Als ganz besonders bevorzugte Pyridiniumionen (IVa) seien genannt 1-Methylpyridinium, 1-Ethylpyridinium, 1-(1-Butyl)pyridinium, 1-(1-Hexyl)pyridinium, 1-(1-Octyl)-pyridinium, 1-(1-Hexyl)-pyridinium, 1-(1-Octyl)-pyridinium, 1-(1-Dodecyl)-pyridinium, 1-(1-Tetradecyl)-pyridinium, 1-(1-Hexadecyl)-pyridinium, 1,2-Dimethylpyridinium, 1-Ethyl-2-methylpyridinium, 1-(1-Butyl)-2-methylpyridinium, 1-(1-Hexyl)-2-methylpyridinium, 1-(1-Octyl)-2-methylpyridinium, 1-(1-Dodecyl)-2-methylpyridinium, 1-(1-Tetradecyl)-2-methylpyridinium, 1-(1-Hexadecyl)-2-methylpyridinium, 1-Methyl-2-ethylpyridinium, 1,2-Diethylpyridinium, 1-(1-Butyl)-2-ethylpyridinium, 1-(1-Hexyl)-2-ethylpyridinium, 1-(1-Octyl)-2-ethylpyridinium, 1-(1-Dodecyl)-2-ethylpyridinium, 1-(1-Tetradecyl)-2-ethylpyridinium, 1-(1-Hexadecyl)-2-ethylpyridinium, 1,2-Dimethyl-5-ethyl-pyridinium, 1,5-Diethyl-2-methyl-pyridinium, 1-(1-Butyl)-2-methyl-3-ethyl-pyridinium, 1-(1-Hexyl)-2-methyl-3-ethyl-pyridinium und 1-(1-Octyl)-2-methyl-3-ethyl-pyridinium, 1-(1-Dodecyl)-2-methyl-3-ethyl-pyridinium, 1-(1-Tetradecyl)-2-methyl-3-ethyl-pyridinium und 1-(1-Hexadecyl)-2-methyl-3-ethyl-pyridinium.

Ganz besonders bevorzugt setzt man als Pyridaziniumionen (IVb) solche ein, bei denen
- R¹ bis R⁴ Wasserstoff sind; oder
- einer der Reste R¹ bis R⁴ Methyl oder Ethyl ist und die verbleibenden Reste R¹ bis R⁴ Wasserstoff sind.

Ganz besonders bevorzugt setzt man als Pyrimidiniumionen (IVc) solche ein, bei denen
- R¹ Wasserstoff, Methyl oder Ethyl ist und R² bis R⁴ unabhängig voneinander Wasserstoff oder Methyl sind; oder
- R¹ Wasserstoff, Methyl oder Ethyl ist, R² und R⁴ Methyl sind und R³ Wasserstoff ist.

Ganz besonders bevorzugt setzt man als Pyraziniumionen (IVd) solche ein, bei denen
- R¹ Wasserstoff, Methyl oder Ethyl ist und R² bis R⁴ unabhängig voneinander Wasserstoff oder Methyl sind;
- R¹ Wasserstoff, Methyl oder Ethyl ist, R² und R⁴ Methyl sind und R³ Wasserstoff ist;
- R¹ bis R⁴ Methyl sind; oder
- R¹ bis R⁴ Wasserstoff sind.

Ganz besonders bevorzugt setzt man als Imidazoliumionen (IVe) solche ein, bei denen
- R¹ Wasserstoff, Methyl, Ethyl, 1-Propyl, 1-Butyl, 1-Pentyl, 1-Hexyl, 1-Octyl, 2-Hydroxyethyl oder 2-Cyanoethyl und R² bis R⁴ unabhängig voneinander Wasserstoff, Methyl oder Ethyl sind.

Als ganz besonders bevorzugte Imidazoliumionen (IVe) seien genannt 1-Methylimidazolium, 1-Ethylimidazolium, 1-(1-Butyl)-imidazolium, 1-(1-Octyl)-imidazolium, 1-(1-Dodecyl)-imidazolium, 1-(1-Tetradecyl)-imidazolium, 1-(1-Hexadecyl)-imidazolium, 1,3-Dimethylimidazolium, 1-Ethyl-3-methylimidazolium, 1-(1-Butyl)-3-methylimidazolium, 1-(1-Butyl)-3-ethylimidazolium, 1-(1-Hexyl)-3-methyl-imidazolium, 1-(1-Hexyl)-3-ethyl-imidazolium, 1-(1-Hexyl)-3-butyl-imidazolium, 1-(1-Octyl)-3-methylimidazolium, 1-(1-Octyl)-3-ethylimidazolium, 1-(1-Octyl)-3-butylimidazolium, 1-(1-Dodecyl)-3-methyl-imidazolium, 1-(1-Dodecyl)-3-ethylimidazolium, 1-(1-Dodecyl)-3-butylimidazolium, 1-(1-Dodecyl)-3-octylimidazolium, 1-(1-Tetradecyl)-3-methylimidazolium, 1-(1-Tetradecyl)-3-ethylimidazolium, 1-(1-Tetradecyl)-3-butylimidazolium, 1-(1-Tetradecyl)-3-octylimidazolium, 1-(1-Hexadecyl)-3-methylimidazolium, 1-(1-Hexadecyl)-3-ethylimidazolium, 1-(1-Hexadecyl)-3-butylimidazolium, 1-(1-Hexadecyl)-3-octylimidazolium, 1,2-Dimethylimidazolium, 1,2,3-Trimethylimidazolium, 1-Ethyl-2,3-dimethylimidazolium, 1-(1-Butyl)-2,3-dimethylimidazolium, 1-(1-Hexyl)-2,3-dimethyl-imidazolium, 1-(1-Octyl)-2,3-dimethylimidazolium, 1,4-Dimethylimidazolium, 1,3,4-Trimethylimidazolium, 1,4-Dimethyl-3-ethylimidazolium, 3-butylimidazolium, 1,4-Dimethyl-3-octylimidazolium, 1,4,5-Trimethylimidazolium, 1,3,4,5-Tetramethylimidazolium, 1,4,5-Trimethyl-3-ethylimidazolium, 1,4,5-Trimethyl-3-butylimidazolium, 1,4,5-Trimethyl-3-octylimidazolium und 1-(Prop-1-en-3-yl)-3-methylimidazolium.

Ganz besonders bevorzugt setzt man als Pyrazoliumionen (IVf), (IVg) beziehungsweise (IVg') solche ein, bei denen
- R¹ Wasserstoff, Methyl oder Ethyl ist und R² bis R⁴ unabhängig voneinander Wasserstoff oder Methyl sind.

Ganz besonders bevorzugt setzt man als Pyrazoliumionen (IVh) solche ein, bei denen
- R¹ bis R⁴ unabhängig voneinander Wasserstoff oder Methyl sind.

Ganz besonders bevorzugt setzt man als 1-Pyrazoliniumionen (IVi) solche ein, bei denen
- unabhängig voneinander R¹ bis R⁶ Wasserstoff oder Methyl sind.

Ganz besonders bevorzugt setzt man als 2-Pyrazoliniumionen (IVj) beziehungsweise (IVj') solche ein, bei denen
- R¹ Wasserstoff, Methyl, Ethyl oder Phenyl ist und R² bis R⁶ unabhängig voneinander Wasserstoff oder Methyl sind.

Ganz besonders bevorzugt setzt man als 3-Pyrazoliniumionen (IVk) beziehungsweise (IVk') solche ein, bei denen
- R¹ und R² unabhängig voneinander Wasserstoff, Methyl, Ethyl oder Phenyl sind und R³ bis R⁶ unabhängig voneinander Wasserstoff oder Methyl sind.

Ganz besonders bevorzugt setzt man als Imidazoliniumionen (IVI) solche ein, bei denen
- R¹ und R² unabhängig voneinander Wasserstoff, Methyl, Ethyl, 1-Butyl oder Phenyl sind, R³ und R⁴ unabhängig voneinander Wasserstoff, Methyl oder Ethyl sind und R⁵ und R⁶ unabhängig voneinander Wasserstoff oder Methyl sind.

Ganz besonders bevorzugt setzt man als Imidazoliniumionen (IVm) beziehungsweise (IVm') solche ein, bei denen
- R¹ und R² unabhängig voneinander Wasserstoff, Methyl oder Ethyl sind und R³ bis R⁶ unabhängig voneinander Wasserstoff oder Methyl sind.

Ganz besonders bevorzugt setzt man als Imidazoliniumionen (IVn) beziehungsweise (IVn') solche ein, bei denen
- R¹ bis R³ unabhängig voneinander Wasserstoff, Methyl oder Ethyl sind und R⁴ bis R⁶ unabhängig voneinander Wasserstoff oder Methyl sind.

Ganz besonders bevorzugt setzt man als Thiazoliumionen (IVo) beziehungsweise (I-Vo') sowie als Oxazoliumionen (IVp) solche ein, bei denen
- R¹ Wasserstoff, Methyl, Ethyl oder Phenyl ist und R² und R³ unabhängig voneinander Wasserstoff oder Methyl sind.

Ganz besonders bevorzugt setzt man als 1,2,4-Triazoliumionen (IVq), (IVq') beziehungsweise (IVq") solche ein, bei denen
- R¹ und R² unabhängig voneinander Wasserstoff, Methyl, Ethyl oder Phenyl sind und R³ Wasserstoff, Methyl oder Phenyl ist.

Ganz besonders bevorzugt setzt man als 1,2,3-Triazoliumionen (IVr), (IVr') beziehungsweise (IVr") solche ein, bei denen
- R¹ Wasserstoff, Methyl oder Ethyl ist und R² und R³ unabhängig voneinander Wasserstoff oder Methyl sind, oder R² und R³ zusammen 1,4-Buta-1,3-dienylen ist.

Ganz besonders bevorzugt setzt man als Pyrrolidiniumionen (IVs) solche ein, bei denen
- R¹ Wasserstoff, Methyl, Ethyl oder Phenyl ist und R² bis R⁹ unabhängig vonein-ander Wasserstoff oder Methyl sind.

Ganz besonders bevorzugt setzt man als Imidazolidiniumionen (IVt) solche ein, bei denen
- R¹ und R⁴ unabhängig voneinander Wasserstoff, Methyl, Ethyl oder Phenyl sind und R² und R³ sowie R⁵ bis R⁸ unabhängig voneinander Wasserstoff oder Methyl sind.

Ganz besonders bevorzugt setzt man als Ammoniumionen (IVu) solche ein, bei denen
- R¹ bis R³ unabhängig voneinander C₁- bis C₁₈-Alkyl sind; oder
- R¹ und R² zusammen 1,5-Pentylen oder 3-Oxa-1,5-pentylen sind und R³ C₁-C₁₈-Alkyl, 2-Hydroxyethyl oder 2-Cyanoethyl ist.

Als ganz besonders bevorzugte Ammoniumionen (IVu) seien genannt Trimethylammonium, Triethylammonium, Dimethylethylammonium, Diethylmethylammonium, Tetramethylammonium.

Beispiele für die tertiären Amine, von denen sich die quatären Ammoniumionen der allgemeinen Formel (IVu) durch Quaternisierung mit den genannten Resten R ableiten, sind Trimethylamin, Triethylamin, Tri-n-propylamin, Tri-n-butylamin, Diethylmethylamin, Dimethylethylamin, Triisopropylamin, Isopropyldiethylamin, Diisopropylethylamin, Diethyl-n-butylamin, Diethyl-tert-butylamin, Diethyl-n-pentylamin, Diethyl-hexylamin, Diethyloctylamin, Diethyl-(2-ethylhexyl)-amin, Di-n-propylbutylamin, Di-n-propyl-n-pentylamin, Di-n-propylhexylamin, Di-n-propyloctylamin, Di-n-propyl-(2-ethyl-hexyl)-amin, Di-isopropylethylamin, Di-iso-propyl-n-propylamin, Di-isopropyl-butylamin, Diisopropylpentylamin, Di-iso-propylhexylamin, Di-isopropyloctylamin, Di-iso-propyl-(2-ethylhexyl)-amin, Di-n-butylethylamin, Di-n-butyl-n-propylamin, Di-n-butyl-n-pentylamin, Di-n-butylhexylamin, Di-n-butyloctylamin, Di-n-butyl-(2-ethylhexyl)-amin, N-n-Butylpyrrolidin, N-sek-Butylpyrrodidin, N-tert-Butylpyrrolidin, N-n-Pentylpyrrolidin, N,N-Dimethylcyclohexylamin, N,N-Diethylcyclohexylamin, N,N-Di-n-butylcyclohexylamin, N-n-Propylpiperidin, N-iso-Propylpiperidin, N-n-Butyl-piperidin, N-sek-Butylpiperidin, N-tert-Butylpiperidin, N-n-Pentylpiperidin, N-n-Butylmorpholin, N-sek-Butylmorpholin, N-tert-Butylmorpholin, N-n-Pentylmorpholin, N-Benzyl-N-ethylanilin, N-Benzyl-N-n-propylanilin, N-Benzyl-N-iso-propylanilin, N-Benzyl-N-n-butylanilin, N,N-Dimethyl-p-toluidin, N,N-Diethyl-p-toluidin, N,N-Di-n-butyl-p-toluidin, Diethylbenzylamin, Di-n-propylbenzylamin, Di-n-butylbenzylamin, Diethylphenylamin, Di-n-Propylphenylamin und Din-Butylphenylamin.

Bevorzugte quartäre Ammoniumsalze der allgemeinen Formel (IVu) sind solche, die sich von folgenden tertiären Aminen durch Quärternisierung mit den genannten Resten R ableiten lassen, wie Dimethylamin, Trimethylamin, Diethylamin, Triethylamin, Dimethylethylamin, Diethyl-tert-butylamin, Di-iso-propylethylamin, Tripropylamin, Tributylamin.

Besonders bevorzugte tertiäre Amine sind Trimethylamin und Triethylamin.

Ganz besonders bevorzugt setzt man als Guanidiniumionen (IVv) solche ein, bei denen
- R¹ bis R⁵ Methyl sind.

Als ganz besonders bevorzugtes Guanidiniumion (IVv) sei genannt N,N,N',N',N",N"-Hexamethylguanidinium.

Ganz besonders bevorzugt setzt man als Choliniumionen (IVw) solche ein, bei denen
- R¹ und R² unabhängig voneinander Methyl, Ethyl, 1-Butyl oder 1-Octyl sind und R³ Wasserstoff, Methyl, Ethyl, Acetyl, -SO₂OH oder -PO(OH)₂ ist;
- R¹ Methyl, Ethyl, 1-Butyl oder 1-Octyl ist, R² eine -CH₂-CH₂-OR⁴-Gruppe ist und R³ und R⁴ unabhängig voneinander Wasserstoff, Methyl, Ethyl, Acetyl, -SO₂OH oder -PO(OH)₂ sind; oder
- R¹ eine -CH₂-CH₂-OR⁴-Gruppe ist, R² eine -CH₂-CH₂-OR⁵-Gruppe ist und R³ bis R⁵ unabhängig voneinander Wasserstoff, Methyl, Ethyl, Acetyl, -SO₂OH oder - PO(OH)₂ sind.

Besonders bevorzugte Choliniumionen (IVw) sind solche, bei denen R³ ausgewählt ist aus Wasserstoff, Methyl, Ethyl, Acetyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Ganz besonders bevorzugt setzt man als Phosphoniumionen (IVx) solche ein, bei denen
- R¹ bis R³ unabhängig voneinander C₁-C₁₈-Alkyl, insbesondere Butyl, Isobutyl, 1-Hexyl oder 1-Octyl sind.

Unter den vorstehend genannten heterocyclischen Kationen sind die Pyridiniumionen, Pyrazolinium-, Pyrazoliumionen und die Imidazolinium- sowie die Imidazoliumionen bevorzugt. Weiterhin sind Ammoniumionen bevorzugt.

Insbesondere bevorzugt sind 1-Methylpyridinium, 1-Ethylpyridinium, 1-(1-Butyl)pyridinium, 1-(1-Hexyl)pyridinium, 1-(1-Octyl)pyridinium, 1-(1-Hexyl)-pyridinium, 1-(1-Octyl)-pyridinium, 1-(1-Dodecyl)-pyridinium, 1-(1-Tetradecyl)-pyridinium, 1-(1-Hexadecyl)-pyridinium, 1,2-Dimethylpyridinium, 1-Ethyl-2-methylpyridinium, 1-(1-Butyl)-2-methylpyridinium, 1-(1-Hexyl)-2-methylpyridinium, 1-(1-Octyl)-2-methylpyridinium, 1-(1-Dodecyl)-2-methylpyridinium, 1-(1-Tetradecyl)-2-methylpyridinium, 1-(1-Hexadecyl)-2-methylpyridinium, 1-Methyl-2-ethylpyridinium, 1,2-Diethylpyridinium, 1-(1-Butyl)-2-ethylpyridinium, 1-(1-Hexyl)-2-ethylpyridinium, 1-(1-Octyl)-2-ethylpyridinium, 1-(1-Dodecyl)-2-ethylpyridinium, 1-(1-Tetradecyl)-2-ethylpyridinium, 1-(1-Hexadecyl)-2-ethylpyridinium, 1,2-Dimethyl-5-ethyl-pyridinium, 1,5-Diethyl-2-methyl-pyridinium, 1-(1-Butyl)-2-methyl-3-ethyl-pyridinium, 1-(1-Hexyl)-2-methyl-3-ethyl-pyridinium, 1-(1-Octyl)-2-methyl-3-ethyl-pyridinium, 1-(1-Dodecyl)-2-methyl-3-ethyl-pyridinium, 1-(1-Tetradecyl)-2-methyl-3-ethyl-pyridinium, 1-(1-Hexadecyl)-2-methyl-3-ethyl-pyridinium, 1-Methylimidazolium, 1-Ethylimidazolium, 1-(1-Butyl)-imidazolium, 1-(1-Octyl)-imidazolium, 1-(1-Dodecyl)-imidazolium, 1-(1-Tetradecyl)-imidazolium, 1-(1-Hexadecyl)-imidazolium, 1,3-Dimethylimidazolium, 1-Ethyl-3-methylimidazolium, 1-(1-Butyl)-3-methylimidazolium, 1-(1-Hexyl)-3-methyl-imidazolium, 1-(1-Octyl)-3-methylimidazolium, 1-(1-Dodecyl)-3-methylimidazolium, 1-(1-Tetradecyl)-3-methylimidazolium, 1-(1-Hexadecyl)-3-methylimidazolium, 1,2-Dimethylimidazolium, 1,2,3-Trimethylimidazolium, 1-Ethyl-2,3-dimethylimidazolium, 1-(1-Butyl)-2,3-dimethylimidazolium, 1-(1-Hexyl)-2,3-dimethyl-imidazolium und 1-(1-Octyl)-2,3-dimethylimidazolium, 1,4-Dimethylimidazolium, 1,3,4-Trimethylimidazolium, 1,4-Dimethyl-3-ethylimidazolium, 3-Butylimidazolium, 1,4-Dimethyl-3-octylimidazolium, 1,4,5-Trimethylimidazolium, 1,3,4,5-Tetramethylimidazolium, 1,4,5-Trimethyl-3-ethylimidazolium, 1,4,5-Trimethyl-3-butylimidazolium, 1,4,5-Trimethyl-3-octylimidazolium und 1-(Prop-1-en-3-yl)-3-metyl-imidazolium.

Bei den in den Formeln (IIIa) bis (IIIj) genannten Metallkationen [M¹]⁺, [M²]⁺, [M³]⁺, [M⁴]²⁺ und [M⁵]³⁺ handelt es sich im Allgemeinen um Metallkationen der 1., 2., 6., 7., 8., 9., 10., 11., 12. und 13. Gruppe des Periodensystems. Geeignete Metallkationen sind beispielsweise Li⁺, Na⁺, K⁺, Cs⁺, Mg²⁺, Ca²⁺, Ba²⁺, Cr³⁺, Fe²⁺, Fe³+, Co²⁺, Ni²⁺, Cu²⁺, Ag⁺, Zn²⁺ und Al³⁺.

Bei dem organischen Kation handelt es sich besonders bevorzugt um ein Ammoniumion, ggf. C₁₋₄-Alkyl-substituiertes Pyridiniumion oder ggf. C₁₋₄-Alkyl-substituiertes Imidazoliumion.

Ganz besonders bevorzugt handelt es sich bei dem organischen Kation um ein Trimethylammoniumion, Triethylammoniumion, unsubstituiertes Pyridiniumion oder 1-Ethyl-3-methylimidazoliumion.

Das Anion der erfindungsgemäß verwendeten ionischen Flüssigkeiten ist ausgewählt unter super-sauren Aluminiumtrichlorid-Lewisbase-Addukten. Aluminiumtrichlorid (AlCl₃) ist eine Lewissäure.

Im Sinne der vorliegenden Erfindung bezeichnet der Ausdruck "super-saure Aluminiumtrichlorid-Lewisbase-Addukte" solche Aluminiumtrichlorid-Lewisbase-Addukte, die in protonierter Form einen pKₛ-Wert aufweisen, welcher kleiner dem einer starken Säure bzw. kleiner oder gleich dem pKₛ-Wert einer überaus starken Säure ist. Bevorzugt weisen die erfindungsgemäß verwendeten super-sauren Aluminiumtrichlorid-Lewisbase-Addukte in protonierter Form einen pKₛ-Wert < -7 auf, d. h. einen kleineren pKₛ-Wert als HCl.

Im Sinne der vorliegenden Erfindung bezeichnet der Ausdruck "Aluminiumtrichlorid-Lewisbase-Addukt" komplexe Anionen, die durch die Anlagerung eines Anions, speziell eines Chlorids oder Bromids, an die Lewissäure Aluminiumtrichlorid gebildet werden. Dabei können die Anlagerungsprodukte auch Addukte mit einem oder zwei weiteren (gleichen oder verschiedenen) Lewissäuremolekülen bilden.

Üblicherweise sind geeignete Lewissäure-Lewisbase-Addukte ausgewählt unter Verbindungen der Formel [MetₐZ_{b}]⁻, worin der Wert von b dem Produkt von Oxidationszahl des Metalls oder Halbmetalls Met und dem Index a, plus 1 entspricht, d. h. b = a•Ox + 1, wobei Ox für die Oxidationszahl des Metalls oder Halbmetalls steht. Üblicherweise weist a einen Wert im Bereich von 1 bis 3 auf. Bevorzugt steht in den Lewissäure-Lewisbase-Addukten a für 2 oder 3.

Wenn a für 2 oder 3 steht, können die im Lewissäure-Lewisbase-Addukt enthaltenen Metalle oder Halbmetalle Met gleich oder verschieden sein. Lewissäure-Lewisbase-Addukte mit verschiedenen Metallen entstehen beispielsweise, wenn sich zuerst ein Lewissäure-Lewisbase-Addukt aus einer Lewissäure und einem Halogenidion bildet und dieses sich anschließend mit einer weiteren, von der ersten Lewissäure verschiedenen Lewissäure unter Adduktbildung umsetzt. Vorzugsweise sind jedoch alle im Lewissäure-Lewisbase-Addukt [MetₐZ_{b}]⁻ enthaltenen Met gleich und zwar Al.

Im Lewissäure-Lewisbase-Addukt der Formel [MetₐZ_{b}]⁻ kann Z gleich oder verschieden sein. Lewissäure-Lewisbase-Addukte mit gemischten Z werden beispielsweise erhalten, wenn, wie oben beschrieben, das Lewissäure-Lewisbase-Addukt aus zwei verschiedenen Lewissäuren entsteht. Alternativ erhält man sie, wenn Lewissäuren mit gemischten Halogenatomen eingesetzt werden oder wenn das Halogenidion, das als Lewisbase fungiert, vom Halogenatom der Lewissäure verschieden ist. Speziell sind alle im Lewissäure-Lewisbase-Addukt der Formel [MetₐZ_{b}]⁻ enthaltenen Z gleich, insbesondere steht Z für Chlor oder Brom.

Beispiele geeigneter Lewisbasen sind Cl⁻, Br, AlCl₄⁻, AlBrCl₃⁻, Al₂Cl₇⁻, Al₂BrCl₆⁻, Al₃Cl₁₀⁻, Al₃BrCl₉⁻, BCl₄⁻, BBr₄⁻, TiCl₅⁻, VCl₆⁻, FeCl₄⁻, FeBr₄⁻, Fe₂Cl₇⁻, Fe₃Cl₁₀⁻, ZnCl₃⁻, ZnBr₃⁻, CuCl₂⁻, CuBr₂⁻, CuCl₃⁻, CuBr₃⁻, NbCl₆⁻, SnCl₃⁻, SnBr₃⁻, SnCl₅⁻, SnBr₅⁻ und (CF₃SO₂)₂N⁻.

Bevorzugte Lewisbasen sind AlCl₄⁻, Al₂Cl₇⁻, BCl₄⁻, BBr₄⁻, TiCl₅⁻, FeCl₄⁻, FeBr₄⁻, Fe₂Cl₇⁻ und Fe₃Cl₁₀⁻.

Dementsprechend ist das Anion der ionischen Flüssigkeit beispielsweise AlCl₄⁻, AlBrCl₃⁻, Al₂Cl₇⁻, Al₂BrCl₆⁻, Al₃Cl₁₀⁻, Al₃BrCl₉⁻ oder (CF₃SO₂)₂NAlCl₃⁻.

Bevorzugte Anionen Y⁻ sind ausgewählt unter AlBrCl₃⁻, Al₂Cl₇⁻, Al₂BrCl₆⁻, AbCl₁₀⁻, Al₃BrCl₉⁻.

Besonders bevorzugt sind die Anionen Y⁻ ausgewählt unter Al₂Cl₇⁻, Al₃Cl₁₀⁻, speziell ist es Al₂Cl₇⁻.

Die Herstellung einer solchen ionischen Flüssigkeit erfolgt insbesondere durch Zugabe der entsprechenden Menge an Aluminiumchlorid zur ionischen Flüssigkeit oder zu einem Ammoniumchlorid.

In der super-sauren ionischen Flüssigkeit, umfassend ein organisches Kation und ein anorganisches Anion, wobei das Anion ein super-saures Aluminiumtrichlorid-Lewisbase-Addukt ist, beträgt das Molverhältnis von Aluminiumtrichlorid zu Lewisbase bevorzugt > 1,0, besonders ≥ 1,5, weiter besonders ≥ 2,0. Das Molverhältnis von Aluminiumtrichlorid zu Lewisbase beträgt bevorzugt ≤ 3,0, besonders ≤ 2,5, ganz besonders bevorzugt = 2,0.

Für die erfindungsgemäß verwendete super-saure ionische Flüssigkeit liegt die Hammett-Funktion Ho bevorzugt im Bereich von -16 bis -20, besonders im Bereich von -17 bis -19.

### Beispiele

### Allgemeine Versuchsdurchführung:

Verwendet wurde jeweils ein 250-ml-Miniplant-Rührbehälter mit Scheibenrührer, Innenthermometer, Intensivkühler, 200-ml-Zulaufgefäß mit Teflonhahn (10 mm Bohrung), Thermometer und Druckausgleich zum Intensivkühler, Rührerantrieb mit Drehzahlanzeige, Probeentnahmeaufsatz mit Hahn und Septum sowie Inertisierung mit über Natriumhydroxid getrocknetem Argon.
Unter Argon wurde die ionische Flüssigkeit (IL) (150 ml) im Rührbehälter vorgelegt und das zu isomerisierende Methylcyclopentan-haltige Organikgemisch (30 ml) in das Zulaufgefäß eingefüllt. In die ionische Flüssigkeit wurde die jeweils angegebene Menge eines Kupfer(II)-Salzes gegeben. Nach Temperierung aller Edukte auf 60 °C wurde unter Rühren durch Öffnen des Teflonhahnes der gesamte Inhalt des Zulaufgefäßes innerhalb von 1-2 Sekunden in den Rührbehälter mit der IL überführt. In vorgegebenen Zeitabständen wurden jeweils 5 ml Probe mittels 30 cm-Kanüle und Spritze über den Probeentnahmestutzen mit Septum entnommen. Nach ca. 2 Minuten wurde die abgeschiedene, leichtere organische Phase aus der Spritze in ca. 5 ml 10 Gew.-%-ige wässrige Natrium-EDTA-Lösung gegeben und geschüttelt. Anschließend wurden die Phasen getrennt. Die organische Phase wurde mit 2 ml Methylenchlorid verdünnt und nach Trocknen mit wasserfreiem Natriumsulfat mittels GC untersucht.
Die folgenden Beispiele betreffen die Isomerisierung von Methylcyclopentan zu Cyclohexan.

### Verwendete Abkürzungen:

RGG: Reaktionsgleichgewicht
MCP: Methylcyclopentan
CH: Cyclohexan
TMA: Trimethylammonium
TEA: Triethylammonium
EMIM: 1-Ethyl-3-methylimidazolium

### Beispiel 1 (Vergleich):

IL: TMA-Al₂Cl₇
Zusatz: keiner
Organik: rein-MCP
Zeit bis zum Erreichen des RGG (80 % MCP-Umsatz): 180 min

### Beispiel 2:

IL: TMA-Al₂Cl₇
Zusatz: 1,22 g CuCl₂
Organik: rein-MCP
Zeit bis zum Erreichen des RGG (80 % MCP-Umsatz): 15 min

### Beispiel 3 (Vergleich):

IL: TMA-Al₂Cl₇
Zusatz: keiner
Organik: 39 Gew.-% MCP, 12 Gew.-% CH, 49 Gew.-% n-Hexan
Zeit bis zum Erreichen des RGG (77 % MCP-Umsatz): 120 min

### Beispiel 4:

IL: TMA-Al₂Cl₇
Zusatz: 0,1 g CuCl₂
Organik: 39 Gew.-% MCP, 12 Gew.-% CH, 49 Gew.-% n-Hexan
Zeit bis zum Erreichen des RGG (77 % MCP-Umsatz): 70 min

### Beispiel 5:

IL: TMA-Al₂Cl₇
Zusatz: 0,5 g CuCl₂
Organik: 39 Gew.-% MCP, 12 Gew.-% CH, 49 Gew.-% n-Hexan
Zeit bis zum Erreichen des RGG (77 % MCP-Umsatz): 30 min

### Beispiel 6:

IL: TMA-Al₂Cl₇
Zusatz: 1,0 g CuCl₂
Organik: 39 Gew.-% MCP, 12 Gew.-% CH, 49 Gew.-% n-Hexan
Zeit bis zum Erreichen des RGG (77 % MCP-Umsatz): 3 min

### Beispiel 7:

IL: TMA-Al₂Cl₇
Zusatz: 0,1 g CuO
Organik: 39 Gew.-% MCP, 12 Gew.-% CH, 49 Gew.-% n-Hexan
Zeit bis zum Erreichen des RGG (77 % MCP-Umsatz): 70 min

### Beispiel 8:

IL: TMA-Al₂Cl₇
Zusatz: 0,5 g CuO
Organik: 39 Gew.-% MCP, 12 Gew.-% CH, 49 Gew.-% n-Hexan
Zeit bis zum Erreichen des RGG (77 % MCP-Umsatz): 30 min

### Beispiel 9:

IL: TMA-Al₂Cl₇
Zusatz: 1,0 g CuO
Organik: 39 Gew.-% MCP, 12 Gew.-% CH, 49 Gew.-% n-Hexan
Zeit bis zum Erreichen des RGG (77 % MCP-Umsatz): 3 min

### Beispiel 10 (Vergleich):

IL: TEA-Al₂Cl₇
Zusatz: keiner
Organik: 39 Gew.-% MCP, 12 Gew.-% CH, 49 Gew.-% n-Hexan
Zeit bis zum Erreichen des RGG (77 % MCP-Umsatz): 60 min

### Beispiel 11:

IL: TEA-Al₂Cl₇
Zusatz: 0,3 g CuO
Organik: 39 Gew.-% MCP, 12 Gew.-% CH, 49 Gew.-% n-Hexan
Zeit bis zum Erreichen des RGG (77 % MCP-Umsatz): 20 min

### Beispiel 12:

IL: TEA-Al₂Cl₇
Zusatz: 1,22 g CuCl₂
Organik: 39 Gew.-% MCP, 12 Gew.-% CH, 49 Gew.-% n-Hexan
Zeit bis zum Erreichen des RGG (77 % MCP-Umsatz): 10 min

### Beispiel 13 (Vergleich):

IL: EMIM-Al₂Cl₇
Zusatz: keiner
Organik: 39 Gew.-% MCP, 12 Gew.-% CH, 49 Gew.-% n-Hexan
RGG (77 % MCP-Umsatz) nach 300 min nicht erreicht, MCP-Umsatz nach 300 min: 70 %

### Beispiel 14:

IL: EMIM-Al₂Cl₂
Zusatz: 1,22 g CuCl₂
Organik: 39 Gew.-% MCP, 12 Gew.-% CH, 49 Gew.-% n-Hexan
Zeit bis zum Erreichen des RGG (80 % MCP-Umsatz): 90 min

### Beispiel 15 (Vergleich):

IL: Pyridinium-Al₂Cl₇
Zusatz: keiner
Organik: 39 Gew.-% MCP, 12 Gew.-% CH, 49 Gew.-% n-Hexan
Zeit bis zum Erreichen des RGG (80 % MCP-Umsatz): 90 min

### Beispiel 16:

IL: Pyridinium-Al₂Cl₇
Zusatz: 1,22 g CuCl₂
Organik: 39 Gew.-% MCP, 12 Gew.-% CH, 49 Gew.-% n-Hexan
Zeit bis zum Erreichen des RGG (80 % MCP-Umsatz): 10 min

### Beispiel 17 (Vergleich):

IL: n-Butylpyridinium-Al₂Cl₇
Zusatz: keiner
Organik: 39 Gew.-% MCP, 12 Gew.-% CH, 49 Gew.-% n-Hexan
RGG (77 % MCP-Umsatz) nach 300 min nicht erreicht, MCP-Umsatz nach 300 min: 67 %

### Beispiel 18:

IL: n-Butylpyridinium-Al₂Cl₇
Zusatz: 1,22 g CuCl₂
Organik: 39 Gew.-% MCP, 12 Gew.-% CH, 49 Gew.-% n-Hexan
Zeit bis zum Erreichen des RGG (80 % MCP-Umsatz): 60 min

## Patentansprüche

1. Verfahren zur Isomerisierung eines gesättigten, verzweigten und zyklischen Kohlenwasserstoffs, wobei bei der Isomerisierung ein tertiäres C-Atom des Kohlenwasserstoffs in ein sekundäres C-Atom umgewandelt wird, **dadurch gekennzeichnet, dass** man die Isomerisierung in Gegenwart einer super-sauren ionischen Flüssigkeit, umfassend ein organisches Kation und ein anorganisches Anion, wobei das Anion ein super-saures Aluminiumtrichlorid-Lewisbase-Addukt ist, und 0,1 bis 1,5 Gew.-% einer Kupfer(II)-Verbindung bezogen auf die eingesetzte super-saure ionische Flüssigkeit durchführt.

2. Verfahren nach Anspruch 1, **dadurch gekennzeichnet, dass** das Molverhältnis von Aluminiumtrichlorid zu Lewisbase > 1,0 bis ≤ 3,0 beträgt.

3. Verfahren nach Anspruch 1, **dadurch gekennzeichnet, dass** das Molverhältnis von Aluminiumtrichlorid zu Lewisbase ≥ 2,0 bis ≤ 2,5 beträgt.

4. Verfahren nach einem der vorhergehenden Ansprüche, **dadurch gekennzeichnet, dass** es sich bei der Kupfer(II)-Verbindung um ein Kupfer(II)-Salz handelt.

5. Verfahren nach einem der vorhergehenden Ansprüche, **dadurch gekennzeichnet, dass** es sich bei der Kupfer(II)-Verbindung um CuCl₂, CuO, CuSO₄, CuBr₂ oder Cul₂ handelt.

6. Verfahren nach einem der Ansprüche 1 bis 5, **dadurch gekennzeichnet, dass** man die Isomerisierung in Gegenwart von 0,5 bis 1,5 Gew.-% der Kupfer(II)-Verbindung, bezogen auf die eingesetzte ionische Flüssigkeit, durchführt.

7. Verfahren nach einem der vorhergehenden Ansprüche, **dadurch gekennzeichnet, dass** es sich bei dem zu isomerisierenden Kohlenwasserstoff um einen C₄₋₁₈-Kohlenwasserstoff handelt.

8. Verfahren nach einem der Ansprüche 1 bis 6, **dadurch gekennzeichnet, dass** es sich bei dem zu isomerisierenden Kohlenwasserstoff um einen C₅₋₈-Kohlenwasserstoff handelt.

9. Verfahren nach einem der vorhergehenden Ansprüche zur Isomerisierung von Methylcyclopentan zu Cyclohexan.

10. Verfahren nach einem der vorhergehenden Ansprüche 1 bis 8 zur Isomerisierung von 1,2-Dimethylcyclopentan, 1,3-Dimethylcyclopentan oder 1,1-Dimethylcyclopentan zu Methylcyclohexan.

11. Verfahren nach einem der vorhergehenden Ansprüche, **dadurch gekennzeichnet, dass** für die super-saure ionische Flüssigkeit enthaltend Aluminiumchlorid die Hammett-Funktion Ho im Bereich von -16 bis -20 liegt.

12. Verfahren nach einem der vorhergehenden Ansprüche, **dadurch gekennzeichnet, dass** es sich bei dem organischen Kation um ein Ammoniumion, ggf. C₁₋₄-Alkylsubstituiertes Pyridiniumion oder ggf. C₁₋₄-Alkyl-substituiertes Imidazoliumion handelt.

13. Verfahren nach einem der vorhergehenden Ansprüche, **dadurch gekennzeichnet, dass** es sich bei dem organischen Kation um ein Trimethylammoniumion, Triethylammoniumion, unsubstituiertes Pyridihiumion oder 1-Ethyl-3-methyl-imidazoliumion handelt.

14. Verfahren nach einem der vorhergehenden Ansprüche, **dadurch gekennzeichnet, dass** es sich bei dem anorganischen Anion um Al₂Cl₇⁻ oder Al₂Cl₆Br⁻ handelt.

15. Verfahren nach einem der vorhergehenden Ansprüche, **dadurch gekennzeichnet, dass** man die Isomerisierung bei einer Temperatur im Bereich von -20 bis 150 °C durchführt.

16. Verfahren nach einem der vorhergehenden Ansprüche, **dadurch gekennzeichnet, dass** man die Isomerisierung bei einem Absolutdruck im Bereich von 1 bis 10 bar durchführt.

17. Verfahren nach einem der vorhergehenden Ansprüche, **dadurch gekennzeichnet, dass** man den zu isomerisierenden Kohlenwasserstoff in einer Konzentration im Bereich von 1 bis 90 Gew.-%, bezogen auf die ionische Flüssigkeit, einsetzt.

## Claims

1. A process for isomerizing a saturated, branched and cyclic hydrocarbon, in which a tertiary carbon atom of the hydrocarbon is converted to a secondary carbon atom in the course of isomerization, which comprises performing the isomerization in the presence of a superacidic ionic liquid comprising an organic cation and an inorganic anion, where the anion is a superacidic aluminium trichloride-Lewis base adduct, and of 0.1 to 1.5% by weight of a copper (II) compound, based on the superacidic ionic liquid used.

2. The process according to claim 1, wherein the molar ratio of aluminium trichloride to Lewis base is > 1.0 to ≤ 3.0.

3. The process according to claim 1, wherein the molar ratio of aluminium trichloride to Lewis base is ≥ 2.0 to ≤ 2.5.

4. The process according to any of the preceding claims, wherein the copper(II) compound is a copper(II) salt.

5. The process according to any of the preceding claims, wherein the copper(II) compound is CuCl₂, CuO, CuSO₄, CuBr₂ or CuI₂.

6. The process according to any of claims 1 to 5, wherein the isomerization is performed in the presence of 0.5 to 1.5% by weight of the copper(II) compound, based on the ionic liquid used.

7. The process according to any of the preceding claims, wherein the hydrocarbon to be isomerized is a C₄₋₁₈ hydrocarbon.

8. The process according to any of claims 1 to 6, wherein the hydrocarbon to be isomerized is a C₅₋₈ hydrocarbon.

9. The process according to any of the preceding claims for isomerizing methylcyclopentane to cyclohexane.

10. The process according to any of the preceding claims 1 to 8 for isomerizing 1,2-dimethylcyclopentane, 1,3-dimethylcyclopentane or 1,1-dimethylcyclopentane to methylcyclohexane.

11. The process according to any of the preceding claims, wherein the Hammett function H₀ for the superacidic ionic liquid comprising aluminium chloride is in the range from -16 to -20.

12. The process according to any of the preceding claims, wherein the organic cation is an ammonium ion, optionally C₁₋₄-alkyl-substituted pyridinium ion or optionally C₁₋₄-alkyl-substituted imidazolium ion.

13. The process according to any of the preceding claims, wherein the organic cation is a trimethylammonium ion, triethylammonium ion, unsubstituted pyridinium ion or 1-ethyl-3-methylimidazolium ion.

14. The process according to any of the preceding claims, wherein the inorganic anion is Al₂Cl₇⁻ or Al₂Cl₆Br⁻.

15. The process according to any of the preceding claims, wherein the isomerization is performed at a temperature in the range from -20 to 150°C.

16. The process according to any of the preceding claims, wherein the isomerization is performed at an absolute pressure in the range from 1 to 10 bar.

17. The process according to any of the preceding claims, wherein the hydrocarbon to be isomerized is used in a concentration in the range from 1 to 90% by weight, based on the ionic liquid.

## Revendications

1. Procédé pour l'isomérisation d'un hydrocarbure saturé, ramifié et cyclique, dans lequel un atome de carbone tertiaire de l'hydrocarbure est converti lors de l'isomérisation en un atome de carbone secondaire, **caractérisé en ce qu'**on effectue l'isomérisation en présence d'un liquide ionique super-acide, comprenant un cation organique et un anion inorganique, l'anion étant un adduit base de Lewis-trichlorure d'aluminium super-acide, et de 0,1 à 1,5 % en poids d'un composé de cuivre(II), par rapport au liquide ionique super-acide utilisé.

2. Procédé selon la revendication 1, **caractérisé en ce que** le rapport molaire du trichlorure d'aluminium à la base de Lewis vaut > 1,0 à ≤ 3,0.

3. Procédé selon la revendication 1, **caractérisé en ce que** le rapport molaire du trichlorure d'aluminium à la base de Lewis vaut ≥ 2,0 à ≤ 2,5.

4. Procédé selon l'une quelconque des revendications précédentes, **caractérisé en ce que** le composé de cuivre(II) consiste en un sel de cuivre(II).

5. Procédé selon l'une quelconque des revendications précédentes, **caractérisé en ce que** le composé de cuivre(II) consiste en CuCl₂, CuO, CuSO₄, CuBr₂ ou CuI₂.

6. Procédé selon l'une quelconque des revendications 1 à 5, **caractérisé en ce qu'**on effectue l'isomérisation en présence de 0,5 à 1,5 % en poids du composé de cuivre(II), par rapport au liquide ionique utilisé.

7. Procédé selon l'une quelconque des revendications précédentes, **caractérisé en ce que** l'hydrocarbure à isomériser consiste en un hydrocarbure en C₄-C₁₈.

8. Procédé selon l'une quelconque des revendications 1 à 6, **caractérisé en ce que** l'hydrocarbure à isomériser consiste en un hydrocarbure en C₅-C₈.

9. Procédé selon l'une quelconque des revendications précédentes, pour l'isomérisation du méthylcyclopropane en cyclohexane.

10. Procédé selon l'une quelconque des revendications 1 à 8, pour l'isomérisation du 1,2-diméthylcyclopentane, du 1,3-diméthylcyclopentane ou du 1,1-diméthylcyclopentane en méthylcyclohexane.

11. Procédé selon l'une quelconque des revendications précédentes, **caractérisé en ce que** pour le liquide ionique super-acide contenant du chlorure d'aluminium la fonction de Hammett H₀ se situe dans la plage de -16 à -20.

12. Procédé selon l'une quelconque des revendications précédentes, **caractérisé en ce que** le cation organique consiste en un ion ammonium, ion pyridinium éventuellement substitué par alkyle en C₁-C₄ ou ion imidazolium éventuellement substitué par alkyle en C₁-C₄.

13. Procédé selon l'une quelconque des revendications précédentes, **caractérisé en ce que** le cation organique consiste en un ion triméthylammonium, ion triéthylammonium, ion pyridinium non substitué ou ion 1-éthyl-3-méthylimidazolium.

14. Procédé selon l'une quelconque des revendications précédentes, **caractérisé en ce que** l'anion inorganique consiste en Al₂Cl₇⁻ ou Al₂Cl₆Br⁻.

15. Procédé selon l'une quelconque des revendications précédentes, **caractérisé en ce qu'**on effectue l'isomérisation à une température dans la plage de -20 à 150 °C.

16. Procédé selon l'une quelconque des revendications précédentes, **caractérisé en ce qu'**on effectue l'isomérisation sous une pression absolue dans la plage de 1 à 10 bars.

17. Procédé selon l'une quelconque des revendications précédentes, **caractérisé en ce qu'**on utilise l'hydrocarbure à isomériser à une concentration dans la plage de 1 à 90% en poids, par rapport au liquide ionique.
